# EUROPÄISCHE PATENTANMELDUNG

(11) **EP 3 809 100 A2**
(43) Veröffentlichungstag der Anmeldung: **21.04.2021**
(21) Anmeldenummer: 20182589.0
(22) Anmeldetag: 26.06.2020
(51) Int. Cl.: G01D 5/26, G01D 11/24

(54) **MULTI-SENOR-KOMPONENTE ZUR BIOPROZESSKONTROLLE**

(30) Priorität: 27.06.2019 DE 102019117446
(71) Anmelder: SCHOTT AG, 55122 Mainz (DE)
(72) Erfinder: OTT, Christian, 84539 Ampfing (DE); KOLBERG, Christoph, 84034 Landshut (DE)
(74) Vertreter: Blumbach · Zinngrebe Patentanwälte PartG mbB

(57) **Zusammenfassung**

Die Erfindung betrifft eine Multi-Sensor-Komponente zur Installation zumindest zweier Sensoren an einem einzelnen Port eines Behälters zur Kultivierung biologischen Materials, insbesondere eines Bioreaktors oder Schüttelkolbens, wobei die Multi-Sensor-Komponente ein Gehäuse aufweist, das zumindest mit einem vorderen Gehäuseabschnitt in eine sich durch den Port des Behälters erstreckende Aufnahmeöffnung einführbar ist, so dass der vordere Gehäuseabschnitt dem Inneren des Behälters zugewandt ist, und wobei die Multi-Sensor-Komponente eine an dem vorderen Gehäuseabschnitt angeordnete erste Sensoreinheit oder eine Halterung für eine erste Sensoreinheit aufweist und eine an dem vorderen Gehäuseabschnitt angeordnete zweite Sensoreinheit oder eine Halterung für eine zweite Sensoreinheit aufweist. Ferner betrifft die Erfindung ein System mit einer Sensoraufnahme und einer darin einführbaren Multi-Sensor-Komponente, einen Behälter mit Port, Multi-Sensor-Komponente und ggf. Sensoraufnahme, eine Luminophoreneinheit zum Anbringen an einer Multi-Sensor-Komponente sowie ein Verfahren zur Herstellung einer Multi-Sensor-Komponente.

## Beschreibung

Die Erfindung betrifft die Prozesskontrolle bei Produktionsprozessen in Behältern zur Kultivierung biologischen Materials, insbesondere Bioreaktoren und Schüttelkolben.

Bioreaktoren und Schüttelkolben dienen zur Kultivierung von Mikroorganismen, tierischen und pflanzlichen Zellen und eröffnen damit ein breites Anwendungsfeld von Biotech-Produktionsprozessen. Generell besteht ein Bedarf, diese Prozesse weiter zu optimieren. Insbesondere für die Herstellung von Biopharmazeutika werden Verbesserungen der Produktausbeute und damit Gewinnsteigerungen forciert. Zur Steuerung und Kontrolle der Produktionsprozesse, Erhöhung der Produktausbeute sowie der Reduktion von Kosten stehen verschiedene Ansätze zur Verfügung.

Einerseits kann eine Steuerung und Kontrolle der Prozesse dadurch erfolgen, dass Substrat- und Produktkonzentrationen ermittelt werden. Dies ist allerdings zeitaufwändig und erfordert in der Regel eine ressourcenintensive Offline-Analytik. Bedingt durch den Probenzug ist hiermit zudem ein nicht unerhebliches Kontaminationsrisiko verbunden.

Andererseits kann die Steuerung der Prozesse und damit die Ausbeute dadurch optimiert werden, dass eine Echtzeit-Prozesskontrolle von Schlüsselparametern durchgeführt wird. Zur Erhöhung der Produktausbeute ist insbesondere das in-situ-Monitoring von Parametern, wie der Temperatur, metabolismus- oder produktbildungsrelevanten Stoffen vorteilhaft, ebenso wie eine Regelung von Kultivierungsbedingungen in Echtzeit.

Zur Echtzeit-Parameterkontrolle können Biosensoren zum Einsatz kommen. Solche Biosensoren können insbesondere an einem Port eines Bioreaktors, eines Schüttelkolbens oder generell jedes Einweg- oder Mehrweg-Behälters zur Kultivierung biologischen Materials installiert werden, denn ein solcher Port bildet eine Öffnung in das Innere des Behälters. Der Port eines Bioreaktors oder Schüttelkolbens entspricht häufig einem bestimmten Standard, so beispielsweise ein Ingold-Port oder ein Broadly-James-Port.

Mitunter ist es auch gewünscht, mehrere Schlüsselparameter zu überwachen. Hierzu kann an einem weiteren Port des Behälters ein weiterer Biosensor installiert werden. Außerdem kann es wünschenswert sein, Parameter mittels spektroskopischer Verfahren zu überwachen, bei denen das Quenching von Luminophoren erfasst wird.

Nachteilig an der parallelen Überwachung mehrerer Parameter kann allerdings ein steigendes Kontaminationsrisiko sein. Außerdem kann, insbesondere bei kleineren Behältern oder bei Schüttelkolben, die Anzahl verfügbarer Ports limitiert sein.

Der Erfindung liegt demnach die Aufgabe zugrunde, eine Echtzeit-Parameterkontrolle mehrerer Parameter unter Reduktion des Kontaminationsrisikos zu ermöglichen und/oder mehrere Parameter auch bei Behältern mit geringer Anzahl verfügbarer Ports zu überwachen.

Diese Aufgabe wird durch die Gegenstände der unabhängigen Ansprüche gelöst. Vorteilhafte Weiterbildungen der Erfindung sind in den abhängigen Ansprüchen definiert.

Erfindungsgemäß wird eine Multi-Sensor-Komponente zur Installation zumindest zweier Sensoren an einem einzelnen Port eines Behälters zur Kultivierung biologischen Materials, insbesondere eines Bioreaktors oder Schüttelkolbens, bereitgestellt.

Die erfindungsgemäße Multi-Sensor-Komponente umfasst ein Gehäuse, das zumindest mit einem vorderen Gehäuseabschnitt in eine sich durch den Port des Behälters erstreckende Aufnahmeöffnung einführbar ist, so dass der vordere Gehäuseabschnitt dem Inneren des Behälters zugewandt ist.

Die sich durch den Port des Behälters erstreckende Aufnahmeöffnung kann beispielsweise durch den Port selbst gebildet sein, d.h. es kann sich um die durch den Port gebildete Durchgangsöffnung in der Behälterwandung handeln, welche das Innere des Behälters mit dem Äußeren des Behälters verbindet. Mit anderen Worten kann die erfindungsgemäße Multi-Sensor-Komponente unmittelbar in den Port einführbar sein.

In einer bevorzugten Ausführungsform ist jedoch vorgesehen, dass es sich bei der durch den Port des Behälters erstreckenden Aufnahmeöffnung um eine Öffnung innerhalb eines in den Port einbringbaren Zwischenelements handelt, insbesondere einer Sensoraufnahme, wie weiter unten noch näher ausgeführt wird. In diesem Fall kann die erfindungsgemäße Multi-Sensor-Komponente beispielsweise unmittelbar in das Zwischenstück bzw. die Sensoraufnahme und damit mittelbar in den Port einführbar sein.

Das Gehäuse der Multi-Sensor-Komponente ist in einer bevorzugten Ausführungsform derart ausgebildet, dass es vollständig in eine sich durch den Port des Behälters erstreckende Aufnahmeöffnung eingeführt werden kann, d.h. im eingesetzten Zustand insbesondere radial vollständig von dem Port oder einer Sensoraufnahme umgeben ist. Das Gehäuse der Multi-Sensor-Komponente kann demnach insbesondere eine entlang der Längsachse der Multi-Sensor-Komponente gleichbleibende Außenform aufweisen, also insbesondere zylindrisch ausgebildet sein, insbesondere entlang der gesamten Länge der Multi-Sensor-Komponente.

Das Gehäuse der erfindungsgemäßen Multi-Sensor-Komponente weist an seinem vorderen Gehäuseabschnitt entweder eine erste Sensoreinheit oder zumindest eine Halterung für eine erste Sensoreinheit auf. Außerdem ist an dem vorderen Gehäuseabschnitt noch eine zweite Sensoreinheit oder zumindest eine Halterung für eine zweite Sensoreinheit angeordnet.

Eine Sensoreinheit ist demnach vorzugsweise an dem vorderen Gehäuseabschnitt angeordnet, also dem Inneren des Behälters zugewandt und insbesondere im Inneren des Behälters befindlich, wenn die Multi-Sensor-Komponente in die sich durch den Port des Behälters erstreckende Aufnahmeöffnung eingeführt ist. Ferner ist eine Halterung für eine Sensoreinheit vorzugsweise an dem vorderen Gehäuseabschnitt angeordnet, also dem Inneren des Behälters zugewandt und insbesondere im Inneren des Behälters befindlich, wenn die Multi-Sensor-Komponente in die sich durch den Port des Behälters erstreckende Aufnahmeöffnung eingeführt ist. Außerdem ist eine Halterung für eine Sensoreinheit vorzugsweise eingerichtet, eine Sensoreinheit derart zu halten, dass diese dem Inneren des Behälters zugewandt und insbesondere im Inneren des Behälters befindlich ist, wenn die Multi-Sensor-Komponente in die sich durch den Port des Behälters erstreckende Aufnahmeöffnung eingeführt ist.

An dem vorderen Gehäuseabschnitt können demnach beispielsweise zwei, insbesondere verschiedene, Sensoreinheiten angeordnet sein. Es kann aber auch vorgesehen sein, dass eine erste Sensoreinheit und eine Halterung für eine zweite, insbesondere verschiedene, Sensoreinheit an dem vorderen Gehäuseabschnitt angeordnet ist. Auch können zwei Halterungen vorgesehen sein, nämlich eine Halterung für eine erste Sensoreinheit sowie eine Halterung für eine zweite, insbesondere verschiedene, Sensoreinheit. Natürlich können auch noch mehr Sensoreinheiten und/oder Halterungen für Sensoreinheiten vorhanden sein.

Mit der Multi-Sensor-Komponente, welche zumindest zwei Sensoreinheiten bzw. Halterungen für Sensoreinheiten umfasst, können in vorteilhafter Weise zwei oder mehr Sensoren in einem Port kombiniert werden, was bei Limitierung der verfügbaren Ports, z.B. bei Schüttelkolben, von Vorteil ist. Insbesondere ermöglicht dies den bedarfsorientierten Einsatz von nicht sterilisierbaren Sensoren, kombiniert in einem Port. Allgemein betrifft die Erfindung sowohl Multi-Use-Anwendungen (Glas- bzw. Edelstahl-Bioreaktoren), Single-Use-Anwendungen (sog. disposable Bioreaktoren bzw. bags) aber auch Schüttelinkubatoren (Schüttelkolben und Zellkulturflaschen).

Vorzugsweise ist zumindest eine Sensoreinheit als Biosensoreinheit, insbesondere zur analytspezifischen Parametermessung, ausgebildet. Mit anderen Worten kann/können die Sensoreinheit, die Sensoreinheiten oder eine der Sensoreinheiten als Biosensoreinheit ausgebildet sein. Eine Biosensoreinheit umfasst vorzugsweise einen Biorezeptor, und ist insbesondere dazu eingerichtet, ein biologisches Signal über den Biorezeptor in ein physiochemisches Signal umzuwandeln. Beispielsweise kann eine Biosensoreinheit dazu ausgebildet sein, eine Konzentration von Sacchariden oder Proteinen zu ermitteln.

Im Fall, dass eine oder zwei Halterungen für eine erste und/oder zweite Sensoreinheit vorgesehen sind, ist alternativ zumindest eine der Halterung zum Halten einer wie vorstehend beschriebenen Biosensoreinheit ausgebildet. Natürlich können auch beide Halterungen dementsprechend ausgebildet sein. Außerdem kann gegebenenfalls auch beides kumulativ vorgesehen sein, beispielsweise ist die erste Sensoreinheit wie vorstehend beschrieben als Biosensoreinheit ausgebildet und die Halterung für die zweite Sensoreinheit ist zum Halten einer wie vorstehend beschriebenen als Biosensoreinheit ausgebildeten Sensoreinheit ausgebildet.

Eine Biosensoreinheit kann als vorzugsweise modularer Baustein ausgebildet sein, beispielsweise in Form eines flachen Chips, und/oder es kann vorgesehen sein, dass eine entsprechende Halterung zum Halten eines solchen Bausteins ausgebildet ist.

Ferner ist vorzugsweise zumindest eine Sensoreinheit, insbesondere eine solche Sensoreinheit, welche nicht als Biosensoreinheit ausgebildet ist, als Luminophoreneinheit zur lumineszenzbasierten Parametermessung ausgebildet. Mit anderen Worten kann/können die Sensoreinheit, die Sensoreinheiten oder eine der Sensoreinheiten als Luminophoreneinheit ausgebildet sein.

Im Fall, dass eine oder zwei Halterungen für eine erste und/oder zweite Sensoreinheit vorgesehen sind, ist alternativ zumindest eine der Halterung zum Halten einer wie vorstehend beschriebenen Luminophoreneinheit ausgebildet. Natürlich können auch beide Halterungen dementsprechend ausgebildet sein. Außerdem kann gegebenenfalls auch beides kumulativ vorgesehen sein, beispielsweise ist die erste Sensoreinheit wie vorstehend beschrieben als Luminophoreneinheit ausgebildet und die Halterung für die zweite Sensoreinheit ist zum Halten einer wie vorstehend beschriebenen als Luminophoreneinheit ausgebildeten Sensoreinheit ausgebildet.

Besonders bevorzugt kann vorgesehen sein, dass die erste Sensoreinheit als Biosensoreinheit ausgebildet ist oder die entsprechende Halterung zum Halten einer Biosensoreinheit ausgebildet ist und die zweite Sensoreinheit als Luminophoreneinheit ausgebildet ist oder die entsprechende Halterung zum Halten einer Luminophoreneinheit ausgebildet ist.

Mit anderen Worten ermöglicht die Erfindung insbesondere den kombinierten Einsatz von Luminophoren und Biosensoren, in dem in einem Gehäuse eine Luminophoreneinheit und eine Biosensoreinheit integriert ist. Die Erfindung betrifft somit eine "Multi-Sensor-Unit" z.B. für die Herstellung von Biopharmazeutika. Hierdurch wird ermöglicht, die Auswertung multipler Parameter (via Luminophore und Biosensoren) in einem Port zu messen.

Eine Sensoreinheit kann auch als Wechselfeldeinheit zur dielektrizitätsbasierten Parametermessung ausgebildet sein. Außerdem kann eine Sensoreinheit auch als Transistoreinheit zur feldeffektbasierten Parametermessung ausgebildet sein. Darüber hinaus kann auch eine Halterung für eine der vorstehenden Sensoreinheiten vorgesehen sein.

Eine Sensoreinheit oder eine entsprechende Halterung kann zumindest eine senkrecht zur Längsrichtung der Multi-Sensor-Komponente verlaufende Abmessung aufweisen, welche größer ist als 35%, vorzugsweise 50%, besonders bevorzugt 65% der Dicke der Multi-Sensor-Komponente senkrecht zu ihrer Längsrichtung. Vorzugsweise kann eine Biosensoreinheit oder eine Halterung für eine Biosensoreinheit eine solche Abmessung aufweisen. Ferner vorzugsweise kann eine Luminophoreneinheit oder eine Halterung für eine Luminophoreneinheit eine solche Abmessung aufweisen. Auch eine Wechselfeldeinheit oder eine Transistoreinheit oder eine Halterung für eine solche Einheit kann eine solche Abmessung aufweisen.

Eine analytspezifische Parametermessung mit einer Biosensoreinheit kann implizieren, dass eine bestimmte Messgröße, der Analyt, selektiv erfasst wird, insbesondere also nicht gleichzeitig mehrere Parameter, z. B. verschiedene Analyten, miterfasst erfasst werden.

Bei der lumineszenbasierten Parametermessung mit einer Luminorphoreneinheit kann ein Analytabhängiges Signalquenching erfolgen. Die Anregungswellenlänge kann durch Wechselwirkung mit der Messgröße wellenlängen- und/oder phasenverschoben werden.

Verschieben sich bei Analyten im elektrischen Feld Ladungen, so wird ein Dipol induziert. Bei einer dielektrizitätsbasierten Messung mit einer Wechselfeldeinheit kann eine frequenzabhängige Wechselwirkung in der Sensoreinheit genutzt werden.

Bei einer feldeffektbasierten Parametermessung mit einer Transistoreinheit können ionische Analyten bei einem Halbleiterelement eine Leitfähigkeit erzeugende Spiegelladung bewirken, insbesondere, indem sie sich ionenselektiv an einen Sensorchip reversibel anlagern.

Eine als Luminophoreneinheit ausgebildete Sensoreinheit umfasst vorzugsweise einen Grundkörper mit einem Aufnahmemittel zur Aufnahme von Luminophorensubstanz, wobei das Aufnahmemittel insbesondere als Kavität in dem Grundkörper ausgebildet ist. Selbstverständlich kann im Fall von ein oder zwei Halterungen wiederum vorgesehen sein, dass eine entsprechende Halterung zum Halten eines solchen Grundkörpers ausgebildet ist.

Der Grundkörper einer Luminophoreneinheit kann einen vorderen und einen hinteren Grundkörperbereich aufweisen, derart dass der vordere Grundkörperbereich dem Inneren des Behälters zugewandt ist und der hintere Grundkörperbereich dem Äußeren des Behälters zugewandt ist, wenn die Luminophoreneinheit in den Port bzw. die sich durch den Port des Behälters erstreckende Aufnahmeöffnung eingeführt ist und/oder wenn die Multi-Sensor-Komponente bzw. ihr vorderer Gehäuseabschnitt in den Port bzw. die sich durch den Port des Behälters erstreckende Aufnahmeöffnung eingeführt ist.

Dabei kann der vordere Grundkörperbereich das zumindest eine, insbesondere als Kavität ausgebildete, Aufnahmemittel umfassen. Alternativ oder zusätzlich kann der hintere Grundkörperbereich zumindest einen Lichtleiter und vorzugsweise eine Lichtquelle (Laser, LED, VCSELs (Vertical-Cavity Surface-Emitting Lasers)) und/oder eine Photodiode umfassen, um Licht einer Lichtquelle an das Aufnahmemittel zu leiten und/oder Licht von dem Aufnahmemittel an eine Photodiode zu leiten.

Der vordere Grundkörperbereich kann aus glasumfassendem Material, insbesondere aus Glas oder Glaskeramik, bestehen oder solches Material umfassen, wobei in dem Material die Aufnahmemittel mittels Laserschneiden als Kavitäten eingebracht sind.

Der hintere Grundkörperbereich kann optische Fasern umfassen oder daraus bestehen, wobei die Fasern vorzugsweise zumindest teilweise den bereits erwähnten Lichtleiter bilden, um Licht an das vorzugsweise als Kavität ausgebildete Aufnahmemittel zu leiten.

In einer bevorzugten Ausführungsform ist der vordere, insbesondere glasumfassende, Grundkörperbereich scheibenförmig ausgebildet und/oder der hintere, vorzugsweise optische Fasern umfassende, Grundkörperbereich stabförmig ausgebildet. Weiter kann der vordere Grundkörperbereich mit dem hinteren Grundkörperbereich durch Laserschweißen verbunden sein.

Grundgerüst der Luminophoreinheit kann somit ein Faserstab sein, wobei dieser am mediumzugewandten bzw. mediumberührenden Ende mit Kavitäten versehen sein kann. Solche Kavitäten können, wie beschrieben, herstellbar sein, indem aus einer Glasscheibe entsprechende Segmente mit einem Laser ausgeschnitten werden und anschließend über Laserschweißen ein Materialverbund mit dem Faserstab hergestellt wird.

Die auf eine jeweilige Luminophor-Kavität ausgerichteten Glasfasern des Faserstabes können über LED- oder Laserlicht angeregt werden. Analyt-konzentrationsabhängig kann die Rate des Quenchings der angeregten Zustände über die entsprechenden Glasfasern vom Luminophor zu den Detektorzellen geleitet werden, wobei beispielsweise im Gehäuse integrierte Photodioden vorgesehen sein können. Durch den Einsatz eines oder mehrerer Lichtleiter können Platzlimitierungen bei der Lichtaus- und -einkopplung kompensierbar sein.

Grundsätzlich ist die Luminophoreneinheit dazu eingerichtet, Luminophorensubstanz aufzunehmen oder zu beherbergen. In einer weiteren Ausführungsform kann diese Substanz aber bereits enthalten sein. Demnach kann vorgesehen sein, dass die Luminophoreneinheit eine Luminophorensubstanz umfasst, wobei die Substanz vorzugsweise durch ein als Kavität ausgebildetes Aufnahmemittel des Grundkörpers beherbergt ist. Die Substanz kann thermisch an oder in dem Aufnahmemittel, z.B. der Kavität, fixiert sein.

Die Aufnahmemittel oder Kavitäten können dazu ausgebildet sein, eine flüssige oder gelartige Luminophorensubstanz aufzunehmen oder eine solche Substanz kann bereits umfasst sein. Die Luminophorensubstanz kann demnach insbesondere als weiche Masse ausgebildet sein (d.h. insbesondere weicher als der Grundkörper und/oder der vordere Grundkörperbereich). Insbesondere bei mehreren Kavitäten können die Luminophore individuell in die Kavitäten gefüllt sein. Dies ermöglicht die Kombination der für eine Prozesskontrolle optimalen Parameter (pH, pO₂, pCO₂, Temperatur, konz. Saccharide, Proteine, etc., wobei Saccharide und Proteine vorzugsweise mit der Biosensoreinheit ermittelbar sind) im gewählten Messbereich in einem Port eines Bioreaktors, Schüttelkolbens oder anderen Behälters mit Port.

Es können demnach bevorzugt mehrere Aufnahmemittel im Grundkörper bzw. vorderen Grundkörperbereich vorgesehen sein, wobei diese jeweils als Kavität ausgebildet sein können, so dass vorteilhaft auch mehrere, insbesondere verschiedene, Luminophorensubstanzen zum Einsatz kommen können.

Die Luminophorensubstanz bzw. der Luminophor umfasst insbesondere Graphen Quantum Dots (GQDs), heterocyclische GQDs (z. B. N-GQDs) und/oder eine metallorganische Verbindung. In Betracht kommen insbesondere:
- DHFAE: 2,7'-dihexyl-5(6)-Noctadecyl-carboxamidofluoresceinethylester und das phosphoreszente Ruthenium(II)-Tris-4,7-diphenyl-1,10-phenanthrolin als inerter Referenzstandard
   pH sensitiver Fluoreszenzfarbstoff für den pH-Bereich 7.3-9.3
- HPTS: 8-Hydroxypyrene-1,3,6-trisulfonische Säure als Trinatriumsalz
   pH sensitiver Fluoreszenzfarbstoff für den pH-Bereich 5.5-8.6 (Zhu et al. 2005)
- PtOEP: Platin« 12, 13, 17, 18-octaethyl-21 H,23H-porphyrin
   pH sensitiver Fluoreszenzfarbstoff
- Pt-PFP: Platin(II)mesotetra(pentafluorophenyl)porphyrin
   O₂ sensitiver Fluoreszenzfarbstoff für den Bereich 0% > O₂ > 21 %
- Ruthenium(II)diimin Komplex: Ruthenium(II)-tris-4,7-diphenyl-1,10-phenanthrolin, mit trimethylsilylpropanesulfonat als Gegenion
   O₂ sensitiver Fluoreszenzfarbstoff für den Bereich 0% > O₂ > 21 %

Aus oder mit einer, insbesondere einer der vorstehend genannten, metallorganischen Verbindung kann mit organisch modifiziertem Silikat ein Xero-Gel hergestellt werden. Applikationsabhängig kann für die Matrix ultrafein gemahlenes Glaspulver bzw. dual-core CoralPor®-Partikel dem Gemisch zugesetzt werden. Die poröse Struktur kann der Verteilung der metallorganischen Substanzen in der Sol-Gel-Matrix dienen. Die Aufkonzentrierung in den Poren kann ein Kriterium bilden, um die Sensorsensitivität und Ansprechzeit zu verbessern. Eine solche Mischung ist ein Luminophor oder eine Luminophorensubstanz und kann durch ein Aufnahmemittel, insbesondere eine Kavität, in dem Grundkörper beherbergt werden. Beispielsweise können die Luminophore für pO₂, pCO₂, pH, Temperatur, etc. jeweils in eine entsprechende Kavität gefüllt und dort thermisch fixiert werden.

Zusätzlich zu den oben genannten metallorganischen Verbindungen kann die Luminophorensubstanz somit ferner auch gemahlenes Glaspulver umfassen. Insbesondere kann diese Substanz mittels eines Sol-Gel-Verfahrens hergestellt oder herstellbar sein und z.B. als ein Xero-Gel zum Einsatz kommen.

Die Multi-Sensor-Komponente kann so ausgebildet sein, dass zumindest eine Sensoreinheit einschiebbar, einklippbar und/oder einrastbar an dem vorderen Gehäuseabschnitt befestigbar ist und/oder dass zumindest eine Halterung zum Einschieben, Einklippen und/oder Einrasten einer Sensoreinheit ausgebildet ist. Ferner kann eine Halterung zum Kontaktieren der Sensoreinheit ausgebildet sein.

Insbesondere die für die jeweilige Kultivierung ausgewählten Biosensoren können somit etwa in Form eines Biosensorchips in eine Führung am Gehäuse einklippbar bzw. kontaktierbar sein.

Insgesamt stellt die beschriebene Multi-Sensor-Komponente somit ein universelles und mannigfaltig adaptierbares System dar. Dieses einzigartige Sensorkonzept ermöglicht es beispielsweise auf Basis einer beschriebenen Multi-Sensor-Komponente und durch Kombination mit verschiedenen metallorganischen Verbindungen und/oder Biosensorchips mannigfaltige Systeme zu realisieren.

Die Bauform ist grundsätzlich für eine universelle Nutzbarkeit für jede Art von Aufnahmeöffnung ausgelegt. Vorzugsweise kann die Bauform für standardisierte Ports (Standard-Stutzen) klassischer Bioreaktoren, für Ports von disposable bzw. Single-Use-Bioreaktoren oder bags oder auch für Schüttelkolbenanwendugen ausgelegt sein.

Bevorzugte Verwendung findet die Multi-Sensor-Unit zusammen mit der Einhaltung von Anforderungen an sterile Kultivierungsbedingungen. Demnach ist in einer bevorzugten Ausführungsform vorgesehen, dass eine sich durch den Port des Behälters erstreckende Sensoraufnahme die Aufnahmeöffnung für die Multi-Sensor-Komponente bildet. Eine solche Sensoraufnahme ist insbesondere dazu eingerichtet, eine Sterilitätsgrenze zwischen Kultivierungsraum und Sensor aufrechtzuerhalten. Ferner kann zur Einhaltung steriler Kultivierungsbedingungen vorgesehen sein, dass die Multi-Sensor-Komponente vor ihrer steril sicheren Aufnahme in eine Aufnahmeöffnung aseptisch vorbehandelt ist oder wird.

Die Erfindung betrifft ferner ein System zur Installation zumindest zweier Sensoren an einem einzelnen Port eines Behälters zur Kultivierung biologischen Materials, insbesondere eines Bioreaktors oder Schüttelkolbens, wobei das System eine Sensoraufnahme und eine Multi-Sensor-Komponente wie vorstehend beschrieben umfasst.

Die Sensoraufnahme weist eine innere Aufnahmeöffnung auf, wobei die Sensoraufnahme zumindest mit einem vorderen Sensoraufnahmeabschnitt in den Port des Behälters einführbar ist, so dass sich die innere Aufnahmeöffnung der Sensoraufnahme durch den Port des Behälters erstreckt.

Die Multi-Sensor-Komponente weist ein Gehäuse auf, das zumindest mit einem vorderen Gehäuseabschnitt in die sich durch den Port des Behälters erstreckende Aufnahmeöffnung der Sensoraufnahme einführbar ist, so dass der vordere Gehäuseabschnitt dem Inneren des Behälters zugewandt ist.

Das Gehäuse der Multi-Sensor-Komponente ist in einer bevorzugten Ausführungsform derart ausgebildet, dass es vollständig in eine sich durch den Port des Behälters erstreckende Aufnahmeöffnung eingeführt werden kann, d.h. im eingesetzten Zustand insbesondere radial vollständig von der Sensoraufnahme umgeben ist. Das Gehäuse der Multi-Sensor-Komponente kann demnach insbesondere eine entlang der Längsachse der Multi-Sensor-Komponente gleichbleibende Außenform aufweisen, also insbesondere zylindrisch ausgebildet sein, insbesondere entlang der gesamten Länge der Multi-Sensor-Komponente.

Die innere Aufnahmeöffnung der Sensoraufnahme ist insbesondere zum Äußeren des Behälters hin geöffnet, so dass die Multi-Sensor-Komponente insbesondere von außen in die Aufnahmeöffnung eingeführt werden kann. Vorzugsweise weist die innere Aufnahmeöffnung entlang ihrer Längsrichtung einen gleichbleibenden Querschnitt auf, ist also insbesondere zylindrisch ausgebildet, insbesondere über einen Teil der Längsrichtung umfassend den nach außen hin geöffneten Endabschnitt der Aufnahmeöffnung.

Bei dem System kann vorgesehen sein, dass die Sensoraufnahme im vorderen Sensoraufnahmeabschnitt geschlossen ausgebildet ist, so dass die innere Aufnahmeöffnung der Sensoraufnahme zum Äußeren des Behälters hin geöffnet und zum Inneren des Behälters hin geschlossen ist, wenn die Sensoraufnahme in den Port des Behälters eingeführt ist.

Der vordere Sensoraufnahmeabschnitt kann zumindest bereichsweise eine offene Porosität aufweisen. Die Porengröße kann z.B. zwischen 40 und 300 Nanometer betragen.

Weiterhin betrifft die Erfindung auch einen Behälter zur Kultivierung biologischen Materials, insbesondere Bioreaktor oder Schüttelkolben, umfassend einen Port, welcher in typischer Weise das Innere des Behälters mit dem Äußeren des Behälters verbindet, sowie eine Multi-Sensor-Komponente und - gegebenenfalls - auch eine Sensoraufnahme.

Die Sensoraufnahme weist eine innere Aufnahmeöffnung auf, wobei die Sensoraufnahme zumindest mit einem vorderen Sensoraufnahmeabschnitt in den Port des Behälters eingeführt ist, so dass sich die innere Aufnahmeöffnung der Sensoraufnahme durch den Port des Behälters erstreckt. Eine Multi-Sensor-Komponente mit einem Gehäuse ist zumindest mit einem vorderen Gehäuseabschnitt in die sich durch den Port des Behälters erstreckende Aufnahmeöffnung der Sensoraufnahme eingeführt, so dass der vordere Gehäuseabschnitt dem Inneren des Behälters zugewandt ist.

Im Fall, dass eine Sensoraufnahme nicht vorgesehen ist, bildet der Port, der das Innere des Behälters mit dem Äußeren des Behälters verbindet, die sich durch den Port erstreckende Aufnahmeöffnung. Die Multi-Sensor-Komponente mit einem Gehäuse ist dann zumindest mit einem vorderen Gehäuseabschnitt in die sich durch den Port des Behälters erstreckende Aufnahmeöffnung eingeführt, so dass der vordere Gehäuseabschnitt dem Inneren des Behälters zugewandt ist.

Die Erfindung betrifft ferner eine Luminophoreneinheit zur luminzeszenzbasierten Parametermessung, insbesondere zum Anbringen an einer Multi-Sensor-Komponente mit einer Halterung zum Halten einer Luminophoreneinheit, wie sie beispielsweise vorstehend beschrieben wurde. Für Details und/oder weitere Ausführungsformen der Luminophoreneinheit gelten die vorstehenden Ausführungen entsprechend.

Weiterhin betrifft die Erfindung noch Verfahren zur Herstellung einer Multi-Sensor-Komponente, wie sie beispielsweise vorstehend beschrieben wurde.

Gemäß einer Verfahrensvariante kann zunächst eine Sensoreinheit, insbesondere eine Luminophoreneinheit, beispielsweise wie vorstehend beschrieben, sowie ein Gehäuse bereitgestellt werden, wobei das bereitgestellte Gehäuse zumindest mit einem vorderen Gehäuseabschnitt in eine sich durch einen Port eines Behälters erstreckende Aufnahmeöffnung einführbar ist, so dass der vordere Gehäuseabschnitt dem Inneren des Behälters zugewandt ist, wobei das bereitgestellte Gehäuse ferner an dem vorderen Gehäuseabschnitt eine Ausnehmung für eine Sensoreinheit, insbesondere eine Luminophoreneinheit, aufweist, und wobei das Gehäuse vorzugsweise ein Polymermaterial, insbesondere Polyetheretherketon (PEEK), umfasst oder aus einem solchen besteht.

In einem weiteren Verfahrensschritt kann dann eines relatives Ausdehnen des Gehäuses gegenüber der Sensoreinheit herbeigeführt werden, insbesondere indem das Gehäuse erwärmt wird. Dann kann die Sensoreinheit in die ausgedehnte Ausnehmung des gegenüber der Sensoreinheit ausgedehnten Gehäuses eingesetzt werden. Damit das Gehäuse die Sensoreinheit in der Ausnehmung fest aufnimmt, kann dann wiederum ein relatives Zusammenziehen des Gehäuses gegenüber der Sensoreinheit herbeigeführt werden, insbesondere indem das Gehäuse abgekühlt wird.

Gemäß einer anderen Verfahrensvariante kann zunächst eine Sensoreinheit, insbesondere eine Luminophoreneinheit, beispielsweise wie vorstehend beschrieben, sowie flüssiges Polymermaterial zur Bildung eines Gehäuses bereitgestellt werden, wobei das bereitgestellte flüssige Polymermaterial insbesondere Polyethylen oder Polypropylen umfasst oder daraus besteht.

In weiteren Verfahrensschritten kann dann das flüssige Polymermaterial von außen an die Sensoreinheit zugeführt werden und zum Aushärten gebracht werden, derart, dass ein die Sensoreinheit fest aufnehmendes Gehäuse gebildet wird.

Nachfolgend werden einige spezielle, nicht abschließend zu verstehende Ausführungsbeispiele der Erfindung Bezug nehmend auf die beigefügten Zeichnungen erläutert. Es zeigen:
- Fig. 1:: eine dreidimensionale Ansicht eines Ports eines Bioreaktors mit Sensoraufnahme,
- Fig. 2:: eine Schnittansicht eines Ports eines Bioreaktors mit Sensoraufnahme, wobei in dieser Schnittansicht Darstellung eine eingeführte Multi-Sensor-Komponente zu sehen ist,
- Fig. 3-5:: weitere dreidimensionale Ansichten eines Ports eines Bioreaktors mit Sensoraufnahme,
- Fig. 6:: eine dreidimensionale Ansicht eines Schüttelkolbens mit einer Schüttelkolben-Kappe mit einem Port,
- Fig. 7:: eine Schnittansicht eines Schüttelkolbens mit Schüttelkolben-Kappe mit einem Port und einer Sensoraufnahme, wobei in dieser Schnittansicht eine eingeführte Multi-Sensor-Komponente zu sehen ist,
- Fig. 8, 9:: weitere dreidimensionale Ansichten eines Schüttelkolbens mit einer Schüttelkolben-Kappe mit einem Port und einer Sensoraufnahme.
- Fig. 10:: eine dreidimensionale Ansicht auf den vorderen Gehäuseabschnitt einer Multi-Sensor-Komponente mit einer Biosensoreinheit und einer Luminophoreneinheit, wobei die Luminophoreneinheit fest im Gehäuse aufgenommen ist,
- Fig. 11:: eine dreidimensionale Ansicht auf den vorderen Gehäuseabschnitt einer Multi-Sensor-Komponente mit einer Biosensoreinheit und einer Luminophoreneinheit, wobei die Luminophoreneinheit wechselbar in einer entsprechenden Halterung in dem Gehäuse befestigt ist,
- Fig. 12:: eine dreidimensionale Ansicht auf den vorderen Gehäuseabschnitt einer Multi-Sensor-Komponente mit einer Halterung für eine Biosensoreinheit und einer Halterung für eine Luminophoreneinheit,
- Fig. 13:: eine dreidimensionale Ansicht auf den hinteren Gehäuseabschnitt einer Multi-Sensor-Komponente mit integrierter Lichtquelle und integrierter Photodiode,
- Fig. 14:: eine dreidimensionale Ansicht auf den hinteren Gehäuseabschnitt einer Multi-Sensor-Komponente, wobei der hintere Grundkörperbereich der Luminophoreneinheit zugänglich ist, um mit einer Lichtquelle und/oder eine Photodiode zu interagieren,
- Fig. 15:: eine dreidimensionale Ansicht einer Biosensoreinheit,
- Fig. 16:: eine dreidimensionale Ansicht einer Luminophoreneinheit mit vorderem und hinterem Grundkörperbereich, und
- Fig. 17:: eine Aufsicht (a) und eine dreidimensionale Ansicht (b) des vorderen Grundkörperbereichs der Luminophoreneinheit aus Fig. 16.

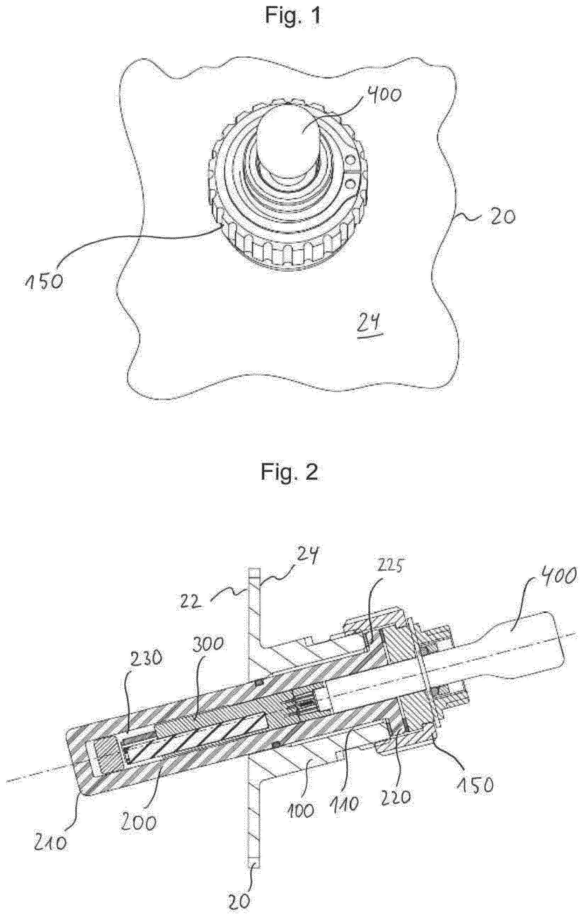

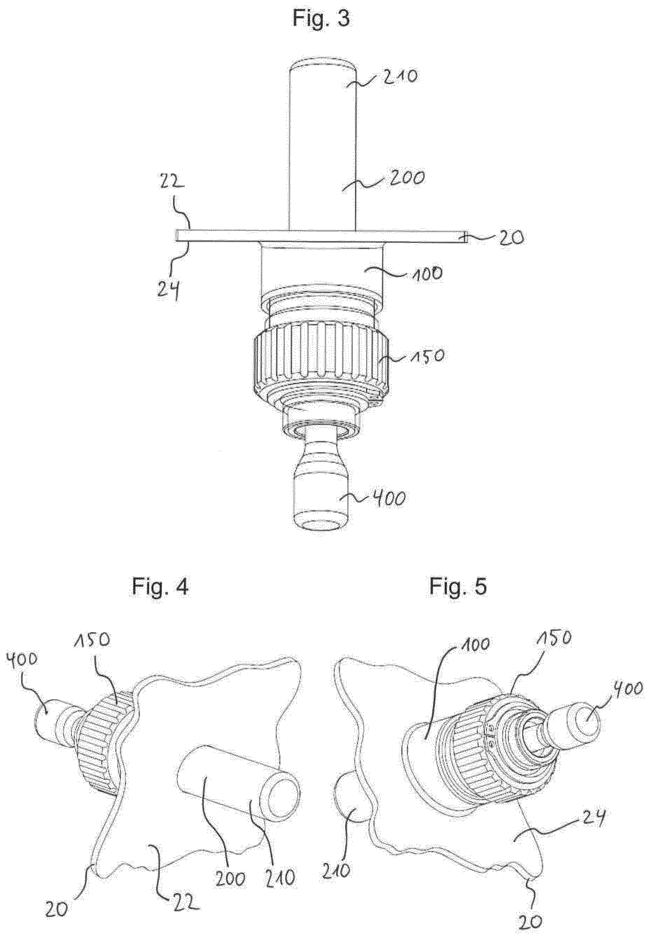

Die Fig.1 - 5 zeigen verschiedene Ansichten eines Ports 100 in einer Wandung 20 eines Bioreaktors, wobei die Wandung 20 des Bioreaktors, welche hier nur bereichsweise dargestellt ist, das Innere des Bioreaktors von dessen Äußerem abgrenzt. In dem hier dargestellten Beispiel handelt es sich um einen, beispielsweise aus Edelstahl gefertigten, Multi-Use-Bioreaktor zur Mehrfachanwendung. Ebenso kann aber auch ein, beispielsweise aus Kunststoff gefertigter, Single-Use-Bioreaktor für Einmalanwendungen oder allgemein ein anderer Behälter mit einem Port 100 zum Einsatz kommen.

In dem hier gezeigten Beispiel ist der in der Wandung 20 des Bioreaktors befindliche Port 100, welcher eine Durchführung durch die Wandung 20 bereitstellt, als Ingold-Port bzw. Ingold-Stutzen ausgebildet. Grundsätzlich kommt aber jede Art von Port 100, der eine Öffnung durch die Wandung 20 bildet, in Betracht. Andere Standardports sind beispielsweise Broadly-James-Ports oder B.-Braun-Sicherheitsports.

Die Wandung 20 des Bioreaktors weist eine nach innen gewandte Innenseite 22 sowie eine nach außen gewandte Außenseite 24 auf. Die Innenseite 22 der Wandung 20 ist der Bioreaktor-Innenseite zugewandt und dem sterilen Bereich zuzurechnen, während die Außenseite 24 der Wandung 20 der Bioreaktor-Außenseite zugewandt ist und dem unsterilen Bereich zuzurechnen ist.

Wie am besten in der Fig. 2 zu sehen ist, ist innerhalb des Ports 100, welcher mit seiner Durchgangsöffnung in das Innere des Behälters zugleich auch eine Aufnahmeöffnung 110 bildet, eine Sensoraufnahme 200 aufgenommen. Die Sensoraufnahme 200 erstreckt sich zumindest teilweise durch die durch den Port 100 gebildete Durchgangsöffnung in der Wandung 20 und ist in dem Port 100 gehalten. In dem gezeigten Beispiel ist die Sensoraufnahme 200 zudem mittels einer Hutmutter 150 lösbar an dem Port 100 arretiert bzw. arretierbar. Die Sensoraufnahme 200 ragt mit einem vorderen Sensoraufnahmeabschnitt 210 in das Innere des Behälters hinein und bei diesem Ausführungsbeispiel ferner mit einem hinteren Sensoraufnahmeabschnitt 220, an welchem sich ein Flansch 225 befindet, zum Äußeren des Behälters hinaus.

Die Sensoraufnahme 200 umfasst eine innere Aufnahmeöffnung 230, welche in dem gezeigten Beispiel zum hinteren, nach außen gerichteten, Sensoraufnahmeabschnitt 220 geöffnet ist. Es kann, wie dargestellt, vorgesehen sein, dass die Aufnahmeöffnung 230 zum vorderen, nach innen gerichteten, Sensoraufnahmeabschnitt 210 hin geschlossen ist. Die Sensoraufnahme 200 wird daher auch als "Steril Port" bezeichnet. Der vordere Sensoraufnahmeabschnitt 210 kann zumindest bereichsweise eine offene Porosität aufweisen.

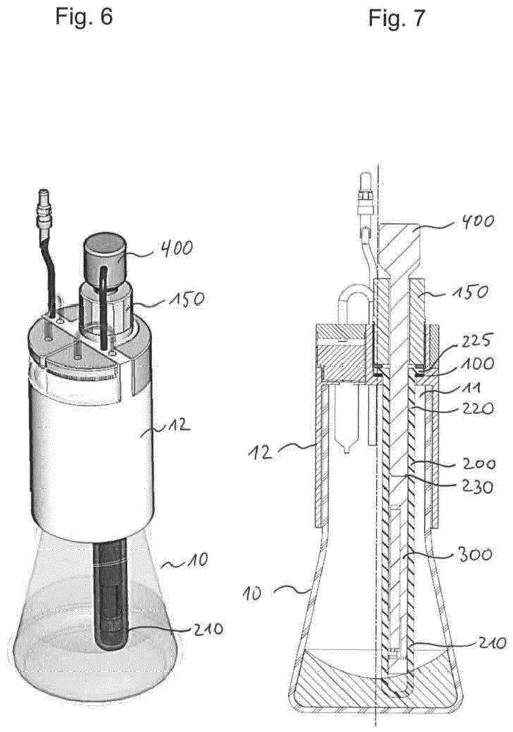

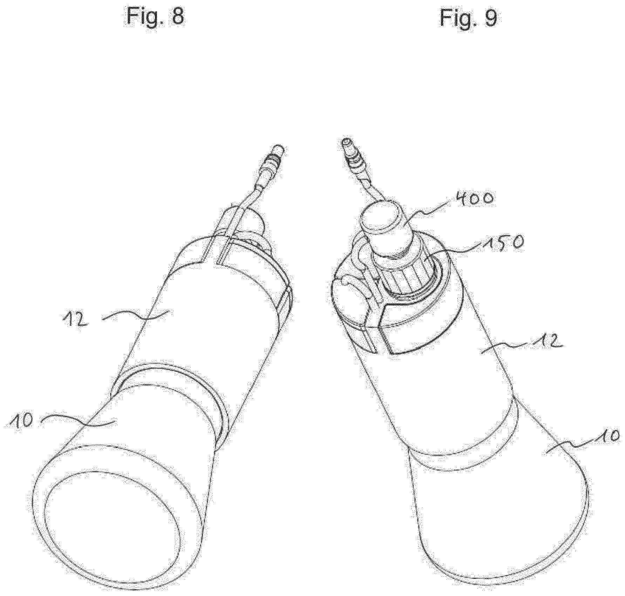

Die Fig. 6 - 9 zeigen verschiedene Ansichten eines Schüttelkolbens 10 mit einer Schüttelkolben-Kappe 12. Die Schüttelkolben-Kappe 12 verschließt die Öffnung 11 des Schüttelkolbens 10 und umfasst einen Port 100 in das Innere des Schüttelkolbens 10. Bei dem Port 100 kann es sich wiederum um jede Art von Durchgangsöffnung handeln, insbesondere um einen standardisierten Port.

Wie am besten in der Fig. 7 zu sehen ist, ist innerhalb des Ports 100, welcher mit seiner Durchgangsöffnung in das Innere des Behälters zugleich auch eine Aufnahmeöffnung 110 bildet, wiederum eine Sensoraufnahme 200 aufgenommen. Die Sensoraufnahme 200 erstreckt sich zumindest teilweise durch den Port 100 und ist darin gehalten. In dem gezeigten Beispiel ist die Sensoraufnahme 200 mittels eines Arretierelements 150 lösbar an dem Port 100 arretiert bzw. arretierbar. Die Sensoraufnahme 200 umfasst wiederum eine innere Aufnahmeöffnung 230, welche zum hinteren Sensoraufnahmeabschnitt 220 geöffnet und zum vorderen Sensoraufnahmeabschnitt 210 hin geschlossen ist.

Bezugnehmend auf Fig. 2 und Fig. 7 befindet sich jeweils innerhalb der Aufnahmeöffnung 230 der Sensoraufnahme 200 eine Multi-Sensor-Komponente 300. In den gezeigten Fällen ist die Multi-Sensor-Komponente 300 vollständig in die Aufnahmeöffnung 230 der Sensoraufnahme 200 eingeführt und mit einem Sondenkopf 400 lösbar verbunden, um die Multi-Sensor-Komponente 300 in die Aufnahmeöffnung 230 einführen und wieder entnehmen zu können. Zwischen dem Sondenkopf 400 und der Multi-Sensor-Komponente 300 kann zudem auch eine elektrische und/oder optische Verbindung bestehen, so dass entsprechende elektrische und/oder optische Signale übertragen werden können.

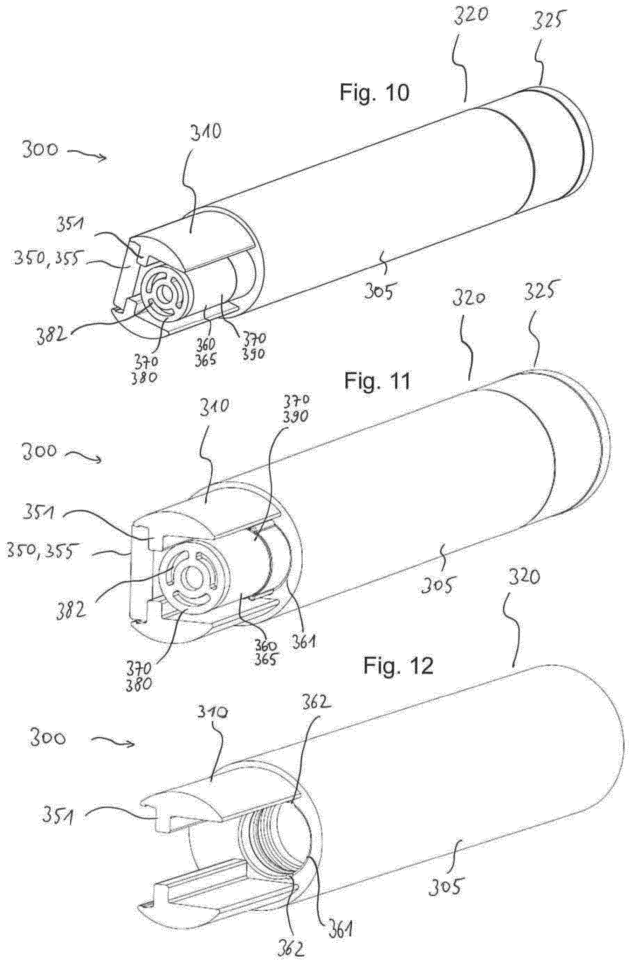

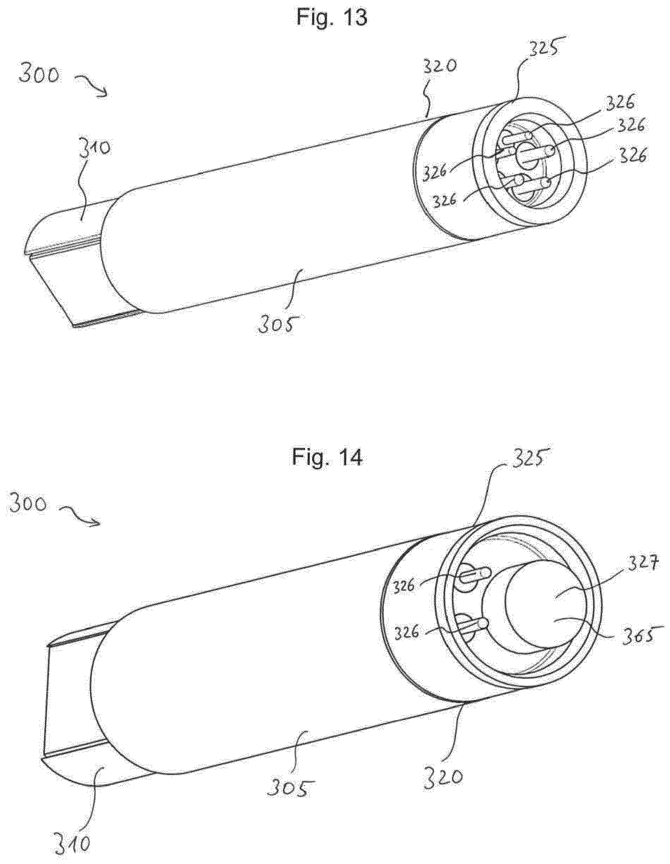

Fig. 10 -14 zeigen verschiedene Ausführungsformen der Multi-Sensor-Komponente 300 im Detail. Die Ausführungsformen der Multi-Sensor-Komponente 300 umfassen jeweils ein Gehäuse 305 mit vorderem Gehäuseabschnitt 310, welcher am besten in den Fig. 10 bis 12 zu sehen ist, und hinterem Gehäuseabschnitt 320, welcher am besten in den Fig. 13 und 14 zu sehen ist.

Am vorderen Gehäuseabschnitt 310 sind eine erste Sensoreinheit 350 und eine zweite Sensoreinheit 360 angeordnet, so dass mit der Multi-Sensor-Komponente 300 zumindest zwei Sensoren an einem einzelnen Port eines Behälters zur Kultivierung biologischen Materials installierbar sind. Der Durchmesser des Gehäuses 305 der Multi-Sensor-Komponente 300 ist so bemessen, dass die Multi-Sensor-Komponente 300 vollständig in eine Aufnahmeöffnung 230 einer Sensoraufnahme 200 einführbar ist.

In dem in Fig. 10 gezeigten Beispiel ist eine der Sensoreinheiten, hier die erste Sensoreinheit 350, als modulare Biosensoreinheit 355 ausgebildet, welche in eine entsprechende Halterung 351 an dem vorderen Gehäuseabschnitt 310 einführbar ist. Zugleich oder unabhängig davon kann eine der Sensoreinheiten als Luminophoreneinheit ausgebildet sein. Hier ist die zweite Sensoreinheit 360 als Luminophoreneinheit 365 ausgebildet, welche am vorderen Gehäuseabschnitt 310 fixiert ist, etwa indem sie von einer Ausnehmung in dem vorderen Gehäuseabschnitt 310 fest aufgenommen ist. Dazu kann insbesondere ein Gehäuse 305 mit oder aus Polyetheretherketon (PEEK) vorgesehen sein, welches z.B. durch Spritzguss oder etwa auch durch ein additives Fertigungsverfahren hergestellt ist, und auf die Luminophoreneinheit 365 aufgeschrumpft ist, also insbesondere zum Ausdehnen und Zusammenziehen gebracht wurde, um die Luminophoreneinheit 365 zu fixieren. Andererseits kann ein Gehäuse 305 mit fest aufgenommener Luminophoreneinheit 365 auch hergestellt sein, indem das Gehäusematerial um die Luminophoreneinheit umspritzt ist.

In dem in Fig. 11 gezeigten Beispiel ist wiederum eine der Sensoreinheiten, hier die erste Sensoreinheit 350, als in einer Halterung 351 gehaltene Biosensoreinheit 355 ausgebildet. Zugleich oder unabhängig davon kann wiederum eine der Sensoreinheiten, hier konkret die zweite Sensoreinheit 360, als Luminophoreneinheit 365 ausgebildet sein. Im Unterschied zu dem in Fig. 10 gezeigten Beispiel ist die Luminophoreneinheit 365 in diesem Beispiel in einer Halterung 361 am vorderen Gehäuseabschnitt 310 gehalten. Die Luminophoreneinheit 365 ist demnach insbesondere wechselbar in dem Gehäuse 305 bzw. der Halterung 361 gehalten.

Das in Fig. 12 gezeigte Ausführungsbeispiel einer Multi-Sensor-Komponente 300 umfasst - wie auch das in Fig. 11 gezeigte Beispiel - ein Gehäuse 305 mit einem vorderen Gehäuseabschnitt 310 und einem hinteren Gehäuseabschnitt 320, wobei an dem vorderen Gehäuseabschnitt 310 eine erste Halterung 351 für eine erste Sensoreinheit 350 sowie eine zweite Halterung 361 für eine zweite Sensoreinheit 360 angeordnet ist. Die erste Halterung 351 ist dabei wiederum zum Halten der Biosensoreinheit 355 ausgebildet. Außerdem kann die zweite Halterung 361 zum Halten einer Luminophoreneinheit 365 ausgebildet sein. Im Unterschied zu dem in Fig. 11 gezeigten Beispiel umfasst die Multi-Sensor-Komponente 300 hier selbst keine Sensoreinheiten.

Der hintere Gehäuseabschnitt 320 einer Multi-Sensor-Komponente 300, welcher am besten in den Fig. 13 und 14 zu sehen ist, kann unabhängig von der Ausgestaltung des vorderen Gehäuseabschnitts 310 unterschiedlich ausgeführt sein. Vorzugsweise befindet sich an dem hinteren Gehäuseabschnitt 320 ein Konnektor 325 mit elektrischen Kontaktelementen 326 und/oder zumindest einem optischen Kontaktelement 327, um eine elektrische und/oder optische Verbindung mit der Multi-Sensor-Komponente und vorzugsweise darin gehaltenen oder befestigten Sensoreinheiten herzustellen.

Der Konnektor 325 der Multi-Sensor-Komponente 300 kann insbesondere dazu ausgelegt sein, mit einem Sondenkopf 400 verbunden zu werden (siehe diesbezüglich z.B. Fig. 2 und Fig. 7). Elektrische Kontaktelemente 326, welche z.B. als Kontaktstifte ausgebildet sein können, sind insbesondere zur Verbindung mit einer Biosensoreinheit 355 oder einer Halterung 351 zum Halten einer Biosensoreinheit 355 vorgesehen. Andererseits können elektrische Kontaktelemente 326 aber auch zur Verbindung mit einer in dem Gehäuse 305 angeordneten Lichtquelle und/oder einem in dem Gehäuse angeordneten Photoelement, z.B. einer Photodiode, ausgelegt sein. Die Multi-Senor-Komponente 300 braucht nicht notwendig eine Lichtquelle bzw. ein Photoelement zum Betrieb der Luminophoreneinheit 365 zu umfassen; vielmehr können diese Komponenten sich auch außerhalb der Multi-Sensor-Komponente 300 befinden. In diesem Fall können ein oder mehrere optische Kontaktelemente 327 vorgesehen sein, wobei es sich dabei insbesondere um Lichtleiter handelt. In einer bevorzugten Ausführungsform weist die Luminophoreneinheit 365 einen, insbesondere stabförmigen als Faserstab ausgebildeten, Lichtleiter auf, welcher in den Konnektor 325 mündet, wie in Fig. 14 zu sehen ist.

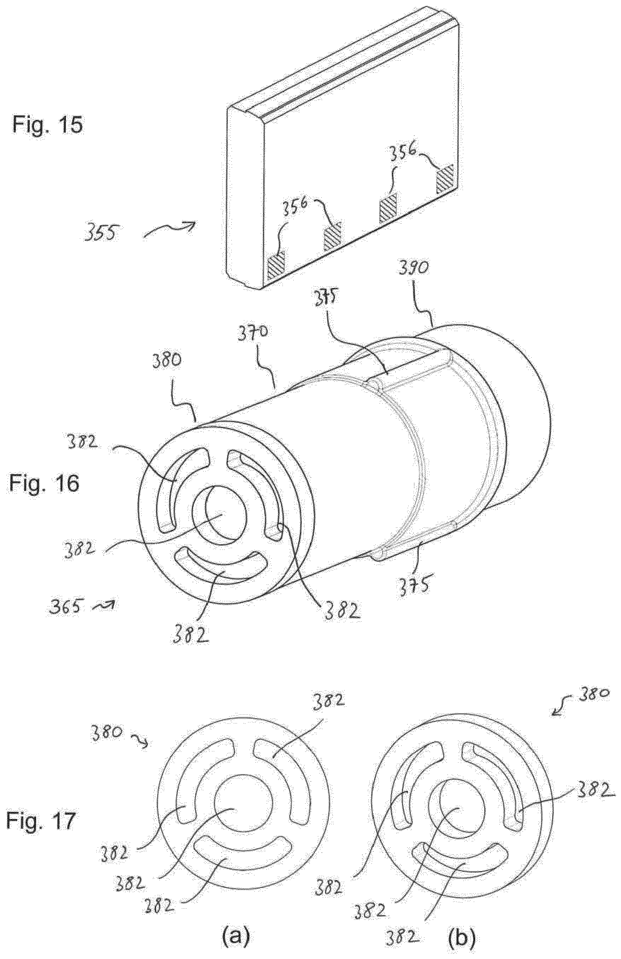

Bezugnehmend auf die Fig. 15 -17 soll nachfolgend auf die Sensoreinheiten, insbesondere die Biosensoreinheit 355 und die Luminophoreneinheit 365, näher eingegangen werden. Obwohl in Fig. 16 eine wechselbare Luminophoreneinheit 365 gezeigt ist, gelten die Ausführungen ebenso für eine fest verbundene Luminophoreneinheit 365, wie sie etwa in Fig. 10 zu sehen ist. Die in Fig. 15 gezeigte Biosensoreinheit 355 hat die Form eines flachen Bausteins, wobei auch andere Bauformen in Betracht kommen. Die Biosensoreinheit 355 ist allerdings vorzugsweise derart geformt, dass sie in eine dafür vorgesehene Halterung 351 eingebracht und von dort wieder entnommen werden kann. Die Biosensoreinheit weist vorzugsweise Kontaktelemente 356 auf, welche als Kontaktflächen ausgebildet sein können, die in der Halterung 351 kontaktiert werden.

Die in Fig. 16 gezeigte Luminophoreneinheit 365 umfasst einen Grundkörper 370 mit einem vorderen Grundkörperbereich 380 und einem hinteren Grundkörperbereich 390. Der vordere Grundkörperbereich 380, welchen Fig. 17 nochmals im Detail zeigt, ist ausgebildet als eine Glasscheibe, in welcher mittels Laserbearbeitung mehrere Kavitäten 382 zu Aufnahme von Luminophorensubstanz eingebracht sind. Der hintere Grundkörperbereich 390 ist als Glasfaserstab ausgebildet, so dass einerseits Licht von einer Lichtquelle an die Kavitäten 382 geleitet werden kann und andererseits Lumineszenzlicht von den Kavitäten wiederum an ein Photoelement, z.B. eine Photodiode, geleitet werden kann. Der Glasfaserstab kann mit der die Kavitäten 382 umfassenden Glasscheibe mittels Laserschweißen verbunden sein. Im Fall einer wechselbaren Luminophoreneinheit 365 kann diese ein z.B. als Führungssteg ausgebildetes Führungselement 375 aufweisen, welches mit einem in der Halterung 361 vorgesehenen, z.B. als Führungsnut ausgebildeten, komplementären Führungselement 362 kooperiert, um die Orientierung der Luminophoreneinheit 365 in ihrer Halterung 362 zu gewährleisten. Solche Führungsstege 375 und Führungsnuten 362 sind in den Fig. 16 bzw. 12 abgebildet.

Insgesamt wird somit ein kombinierter Einsatz von Luminophoren und Biosensoren ermöglicht, in dem in einem Gehäuse 305 eine Luminophoreinheit 365 und eine Biosensoreinheit 355 integriert ist. Luminophore können hierbei individuell in die Kavitäten 382 gefüllt werden. Eine solche Multi-Sensor-Komponente ermöglicht die für die Prozesskontrolle optimalen Parameter wie pH, pO₂, pCO₂, Temperatur, Konzentration von Sacchariden, Proteinen, Ionen, Impedanz für Zellwachstum etc., im gewählten Messbereich in einem Port zu kombinieren, während ein alleiniger Einsatz von Luminophoren an einem Port (oder ein alleiniger Einatz von Biosensoren an einem Port) keine Prozesskontrolle all dieser Parameter ermöglichen würde.

## Patentansprüche

1. Multi-Sensor-Komponente (300) zur Installation zumindest zweier Sensoren an einem einzelnen Port (100) eines Behälters zur Kultivierung biologischen Materials, insbesondere eines Bioreaktors oder Schüttelkolbens, umfassend:
ein Gehäuse (305), das zumindest mit einem vorderen Gehäuseabschnitt (310) in eine sich durch den Port (100) des Behälters erstreckende Aufnahmeöffnung (110) einführbar ist, so dass der vordere Gehäuseabschnitt (310) dem Inneren des Behälters zugewandt ist,
eine an dem vorderen Gehäuseabschnitt (310) angeordnete erste Sensoreinheit (350) oder eine Halterung (351) für eine erste Sensoreinheit (350) und
eine an dem vorderen Gehäuseabschnitt (310) angeordnete zweite Sensoreinheit (360) oder eine Halterung (361) für eine zweite Sensoreinheit (360).

2. Multi-Sensor-Komponente (300) nach Anspruch 1, wobei
zumindest eine Sensoreinheit (351) als Biosensoreinheit (355), insbesondere zur analytspezifischen Parametermessung, ausgebildet ist,
oder zumindest eine Halterung (351) zum Halten einer solchen Biosensoreinheit (355) ausgebildet ist.

3. Multi-Sensor-Komponente (300) nach Anspruch 2, wobei
eine Biosensoreinheit (355) als vorzugsweise modularer Baustein ausgebildet ist, beispielsweise in Form eines flachen Chips,
oder eine entsprechende Halterung (351) zum Halten eines solchen Bausteins ausgebildet ist.

4. Multi-Sensor-Komponente (300) nach einem der Ansprüche 1 bis 3, wobei
zumindest eine Sensoreinheit (350), welche insbesondere nicht als Biosensoreinheit (355) ausgebildet ist, als Luminophoreneinheit (365) zur luminzeszenzbasierten Parametermessung ausgebildet ist
oder wobei zumindest eine Halterung (361), welche nicht zum Halten einer Biosensoreinheit (355) ausgebildet ist, zum Halten einer solchen Luminophoreneinheit (365) ausgebildet ist.

5. Multi-Sensor-Komponente (300) nach Anspruch 4, wobei
eine Sensoreinheit (360), welche als Luminophoreneinheit (365) ausgebildet ist, einen Grundkörper (370) mit einem Aufnahmemittel zur Aufnahme von Luminophorensubstanz umfasst, wobei das Aufnahmemittel insbesondere als Kavität (382) in dem Grundkörper (370) ausgebildet ist,
oder wobei eine entsprechende Halterung (351) zum Halten eines solchen Grundkörpers (370) ausgebildet ist.

6. Multi-Sensor-Komponente (300) nach Anspruch 5, wobei
der Grundkörper (370) der Luminophoreneinheit (365) einen vorderen (380) und einen hinteren Grundkörperbereich (390) aufweist, derart dass der vordere Grundkörperbereich (380) dem Inneren des Behälters zugewandt ist und der hintere Grundkörperbereich (390) dem Äußeren des Behälters zugewandt ist, wenn die Multi-Sensor-Komponente (300) zumindest mit dem vorderen Gehäuseabschnitt (310) in die sich durch den Port (100) des Behälters erstreckende Aufnahmeöffnung (110) eingeführt ist, wobei
der vordere Grundkörperbereich (380) der Luminophoreneinheit (365) das zumindest eine, insbesondere als Kavität (382) ausgebildete, Aufnahmemittel umfasst und
der hintere Grundkörperbereich (390) der Luminophoreneinheit (365) zumindest einen Lichtleiter und vorzugsweise eine Lichtquelle und/oder ein Photoelement umfasst, um Licht von der oder einer Lichtquelle an das Aufnahmemittel zu leiten und/oder Licht von dem Aufnahmemittel an das oder ein Photoelement zu leiten.

7. Multi-Sensor-Komponente (300) nach Anspruch 6, wobei
der vordere Grundkörperbereich (380) aus glasumfassendem Material, insbesondere aus Glas oder Glaskeramik, besteht oder solches Material umfasst, wobei darin die Aufnahmemittel vorzugsweise mittels Laserschneiden als Kavitäten (382) eingebracht sind.

8. Multi-Sensor-Komponente (300) nach einem der Ansprüche 6 oder 7, wobei
der hintere Grundkörperbereich (390) optische Fasern umfasst oder daraus besteht, welche vorzugsweise zumindest teilweise den Lichtleiter bilden, um Licht an das, insbesondere als Kavität (382) ausgebildete, Aufnahmemittel zu leiten.

9. Multi-Sensor-Komponente (300) nach einem der Ansprüche 6 bis 8, wobei
der vordere, insbesondere glasumfassende, Grundkörperbereich (380) scheibenförmig ausgebildet ist,
der hintere, insbesondere optische Fasern umfassende, Grundkörperbereich (390) stabförmig ausgebildet ist und/oder
der vordere Grundkörperbereich (380) mit dem hinteren Grundkörperbereich (390) durch Laserschweißen verbunden ist.

10. Multi-Sensor-Komponente (300) nach einem der Ansprüche 4 bis 8, wobei
die Luminophoreneinheit (365) eine Luminophorensubstanz umfasst, vorzugsweise beherbergt durch ein als Kavität (382) ausgebildetes Aufnahmemittel des Grundkörpers (370).

11. Multi-Sensor-Komponente (300) nach Anspruch 10, wobei
die Luminophorensubstanz Graphen Quantum Dots (GQDs), heterocyclische GQDs (z. B. N-GQDs) und/oder eine metallorganische Verbindung umfasst, ferner vorzugsweise gemahlenes Glaspulver umfasst, und
wobei die Luminophorensubstanz vorzugsweise mittels eines Sol-Gel-Verfahrens hergestellt oder herstellbar ist, insbesondere als Xero-Gel.

12. Multi-Sensor-Komponente (300) nach einem der vorstehenden Ansprüche, wobei
zumindest eine Sensoreinheit einschiebbar, einklippbar und/oder einrastbar an dem vorderen Gehäuseabschnitt (310) befestigt ist
oder eine Halterung (351) zum Einschieben, Einklippen und/oder Einrasten einer Sensoreinheit ausgebildet ist.

13. System zur Installation zumindest zweier Sensoren an einem einzelnen Port (100) eines Behälters zur Kultivierung biologischen Materials, insbesondere eines Bioreaktors oder Schüttelkolbens, umfassend:
eine Sensoraufnahme (200) mit einer inneren Aufnahmeöffnung (230), wobei die Sensoraufnahme (200) zumindest mit einem vorderen Sensoraufnahmeabschnitt in den Port (100) des Behälters einführbar ist, so dass sich die innere Aufnahmeöffnung (230) der Sensoraufnahme (200) durch den Port (100) des Behälters erstreckt und
eine Multi-Sensor-Komponente (300), insbesondere nach einem der Ansprüche 1 bis 12, mit einem Gehäuse (305) das zumindest mit einem vorderen Gehäuseabschnitt (310) in die sich durch den Port (100) des Behälters erstreckende Aufnahmeöffnung (110) der Sensoraufnahme (200) einführbar ist, so dass der vordere Gehäuseabschnitt (310) dem Inneren des Behälters zugewandt ist.

14. System nach Anspruch 13, wobei die Sensoraufnahme (200) im vorderen Sensoraufnahmeabschnitt geschlossen ausgebildet ist, so dass die innere Aufnahmeöffnung (230) der Sensoraufnahme (200) zum Äußeren des Behälters hin geöffnet und zum Inneren des Behälters hin geschlossen ist, wenn die Sensoraufnahme (200) in den Port (100) des Behälters eingeführt ist.

15. System nach Anspruch 14, wobei
der vordere Sensoraufnahmeabschnitt zumindest bereichsweise eine offene Porosität aufweist.

16. Behälter zur Kultivierung biologischen Materials, insbesondere Bioreaktor oder Schüttelkolben, umfassend
einen Port (100), der das Innere des Behälters mit dem Äußeren des Behälters verbindet,
eine Sensoraufnahme (200) mit einer inneren Aufnahmeöffnung (110), wobei die Sensoraufnahme (200) zumindest mit einem vorderen Sensoraufnahmeabschnitt in den Port (100) des Behälters eingeführt ist, so dass sich die innere Aufnahmeöffnung (230) der Sensoraufnahme (200) durch den Port (100) des Behälters erstreckt und
eine Multi-Sensor-Komponente (300), insbesondere nach einem der Ansprüche 1 bis 12, mit einem Gehäuse (305) das zumindest mit einem vorderen Gehäuseabschnitt (310) in die sich durch den Port (100) des Behälters erstreckende Aufnahmeöffnung (230) der Sensoraufnahme (200) eingeführt ist, so dass der vordere Gehäuseabschnitt (310) dem Inneren des Behälters zugewandt ist.

17. Behälter zur Kultivierung biologischen Materials, insbesondere Bioreaktor oder Schüttelkolben, umfassend
einen Port (100), der das Innere Behälters mit dem Äußeren des Behälters verbindet und eine sich durch den Port (100) erstreckende Aufnahmeöffnung (110) bildet und
eine Multi-Sensor-Komponente (300), insbesondere nach einem der Ansprüche 1 bis 12, mit einem Gehäuse (305), das zumindest mit einem vorderen Gehäuseabschnitt (310) in die sich durch den Port (100) des Behälters erstreckende Aufnahmeöffnung (110) eingeführt ist, so dass der vordere Gehäuseabschnitt (310) dem Inneren des Behälters zugewandt ist.

18. Luminophoreneinheit (365) zur lumineszenzbasierten Parametermessung, insbesondere zum Anbringen an einer Multi-Sensor-Komponente (300) mit einer Halterung (361) zum Halten einer Luminophoreneinheit (365), insbesondere nach einem der Ansprüche 1 bis 12.

19. Luminophoreneinheit (365) nach Anspruch 18, umfassend einen Grundkörper (370) mit einem Aufnahmemittel zur Aufnahme von Luminophorensubstanz, wobei das Aufnahmemittel insbesondere als Kavität (382) in dem Grundkörper (370) ausgebildet ist.

20. Luminophoreneinheit (365) nach Anspruch 18 oder 19, wobei der Grundkörper (370) einen vorderen (380) und einen hinteren Grundkörperbereich (390) aufweist,
wobei der vordere Grundkörperbereich (380) das zumindest eine, insbesondere als Kavität (382) ausgebildete, Aufnahmemittel umfasst und
der hintere Grundkörperbereich (390) zumindest einen Lichtleiter und vorzugsweise eine Lichtquelle und/oder ein Photoelement umfasst, um Licht von der oder einer Lichtquelle an das Aufnahmemittel zu leiten und/oder Licht von dem Aufnahmemittel an das oder ein Photoelement zu leiten.

21. Luminophoreneinheit (365) nach einem der Ansprüche 18 bis 20, wobei
der vordere Grundkörperbereich (380) aus glasumfassendem Material, insbesondere aus Glas oder Glaskeramik, besteht oder solches Material umfasst, wobei darin die Aufnahmemittel vorzugsweise mittels Laserschneiden als Kavitäten (382) eingebracht sind.

22. Luminophoreneinheit (365) nach einem der Ansprüche 18 bis 21, wobei
der hintere Grundkörperbereich (390) optische Fasern umfasst oder daraus besteht, welche vorzugsweise zumindest teilweise den Lichtleiter bilden, um Licht an das, insbesondere als Kavität (382) ausgebildete, Aufnahmemittel zu leiten.

23. Luminophoreneinheit (365) nach einem der Ansprüche 18 bis 22, wobei
der vordere, insbesondere glasumfassende, Grundkörperbereich scheibenförmig ausgebildet ist,
der hintere, insbesondere optische Fasern umfassende, Grundkörperbereich stabförmig ausgebildet ist und/oder
der vordere Grundkörperbereich (380) mit dem hinteren Grundkörperbereich (390) durch Laserschweißen verbunden ist.

24. Luminophoreneinheit (365) nach einem der Ansprüche 18 bis 23, wobei
die Luminophoreneinheit (365) eine Luminophorensubstanz umfasst, vorzugsweise beherbergt durch ein als Kavität (382) ausgebildetes Aufnahmemittel des Grundkörpers (370).

25. Luminophoreneinheit (365) nach Anspruch 24, wobei
die Luminophorensubstanz Graphen Quantum Dots (GQDs), heterocyclische GQDs (z. B. N-GQDs) und/oder eine metallorganische Verbindung umfasst, ferner vorzugsweise gemahlenes Glaspulver umfasst, und
wobei die Luminophorensubstanz vorzugsweise mittels eines Sol-Gel-Verfahrens hergestellt oder herstellbar ist, insbesondere als Xero-Gel.

26. Verfahren zur Herstellung einer Multi-Sensor-Komponente (300), insbesondere nach einem der Ansprüche 1 bis 12, umfassend
Bereitstellen einer Sensoreinheit, insbesondere einer Luminophoreneinheit (365), insbesondere nach einem der Ansprüche 18 bis 25,
Bereitstellen eines Gehäuses (305), das zumindest mit einem vorderen Gehäuseabschnitt (310) in eine sich durch einen Port (100) eines Behälters erstreckende Aufnahmeöffnung (110) einführbar ist, so dass der vordere Gehäuseabschnitt (310) dem Inneren des Behälters zugewandt ist,
wobei das bereitgestellte Gehäuse (305) an dem vorderen Gehäuseabschnitt (310) eine Ausnehmung für eine Sensoreinheit, insbesondere eine Luminophoreneinheit (365), aufweist, und wobei das Gehäuse (305) vorzugsweise ein Polymermaterial, insbesondere Polyetheretherketon (PEEK), umfasst oder aus einem solchen besteht,
Herbeiführen eines relativen Ausdehnens des Gehäuses (305) gegenüber der Sensoreinheit, insbesondere durch Erwärmen des Gehäuses (305),
Einsetzen der Sensoreinheit in die Ausnehmung des gegenüber der Sensoreinheit ausgedehnten Gehäuses (305),
Herbeiführen eines relativen Zusammenziehens des Gehäuses (305) gegenüber der Sensoreinheit, insbesondere durch Abkühlen des Gehäuses (305), so dass das Gehäuse (305) die Sensoreinheit in der Ausnehmung fest aufnimmt.

27. Verfahren zur Herstellung einer Multi-Sensor-Komponente (300), insbesondere nach einem der Ansprüche 1 bis 12, umfassend
Bereitstellen einer Sensoreinheit, insbesondere einer Luminophoreneinheit (365), insbesondere nach einem der Ansprüche 18 bis 25,
Bereitstellen flüssigen Polymermaterials zur Bildung eines Gehäuses (305), wobei das bereitgestellte flüssige Polymermaterial insbesondere Polyethylen oder Polypropylen umfasst oder daraus besteht,
Zuführen des flüssigen Polymermaterials von außen an die Sensoreinheit und Aushärten des flüssigen Polymermaterials, derart dass ein die Sensoreinheit fest aufnehmendes Gehäuse (305) gebildet wird, welches zumindest mit einem vorderen Gehäuseabschnitt (310) in eine sich durch einen Port (100) eines Behälters erstreckende Aufnahmeöffnung (110) einführbar ist, so dass der vordere Gehäuseabschnitt (310) dem Inneren des Behälters zugewandt ist.
